# EUROPEAN PATENT APPLICATION

(11) **EP 4 052 669 A1**
(43) Date of publication of application: **07.09.2022**
(21) Application number: 21160837.7
(22) Date of filing: 04.03.2021
(51) Int. Cl.: A61B 17/34, G01C 9/00

(54) **GRAVITY-RESPONSIVE INCLINOMETER FOR A MEDICAL DEVICE**

(71) Applicant: 25Segments AG, 8008 Zürich (CH)
(72) Inventor: FARSHAD, Mazda, 8126 Zumikon (CH); FÜRNSTAHL, Philipp, 8193 Eglisau (CH)
(74) Representative: Liebetanz, Michael

(57) **Abstract**

A gravity-responsive inclinometer (100) for a medical device, especially an injection needle and syringe assembly, has a longitudinal coupling portion (10) defining a predetermined device angle direction (15), wherein the longitudinal coupling portion (10) comprises a connector part (11) configured to be removably connected to the medical device, an at least partially transparent recipient (20), a ball (30) and a measurement scale (25, 26, 27), wherein the ball (30) is moving in the transparent recipient (20), indicating an angle vis-à-vis the predetermined angle direction (15) through its position visible on the measurement scale (25, 26, 27). The transparent recipient (20) is a hollow (22) transparent spherical cap (21) attached (13) at the connector part (11) and the measurement scale (25) comprises an indicator grid (26, 27).

## Description

### TECHNICAL FIELD

The present invention relates to a gravity-responsive inclinometer for a medical device, especially an injection needle and syringe assembly, having a longitudinal coupling portion defining a predetermined device angle direction, wherein the longitudinal coupling portion comprises a connector part configured to be removably connected to the medical device, an at least partially transparent recipient, a ball and a measurement scale, wherein the ball is moving in the transparent recipient, indicating an angle vis-à-vis the predetermined angle direction through its position visible on the measurement scale.

### PRIOR ART

The anesthesiologist, radiologist or surgeon is frequently required to inject nerves with a local anesthetic solution or a neurolytic solution for nerve blockade. A successful nerve block depends on correct placement of the injecting needle. In particular, many blocks of nerves near skeletal structures require precise insertion of the injecting needle; that is, the needle must be inserted at a specific angle to the skin so that the needle is directed toward the required nerve.

For example, in caudal epidural anesthesia the needle is inserted into the caudal canal at an angle of 30 to 45 degrees in the female and 10 to 20 degrees in the male. In intercostal nerve blocks the needle forms an 80 degree angle with the skin. In a celiac plexus block the needle is introduced at an angle of 45 degrees and increased to 60 degrees when the needle contacts the lumbar vertebra. In a paravertebral lumbar sympathetic ganglion block the needle is inserted at an angle of 45 degrees to the skin, and, when the transverse process is contacted, the angle is changed to 85 degrees. Another example is facet joint injection of the lumbar spine where the needle is advanced perpendicularly to the joint until its tip is in the joint.

The measurement of one or multiple angles is also a requirement within a surgery. For instance, in a periacetabular osteotomy, the acetabulum of the pelvis is mobilized through an osteotomy and reoriented three-dimensionally based on pre-defined angels, wherein these pre-defines angles can stem from preoperative image data. The correct reorientation is assessed either by eyeball or by using technology (imaging, surgical navigation).

Prior art provides a number of devices to be used in a syringe and injection needle assembly, e.g. US 4,479,800 A discloses a syringe and injection needle assembly with a gravity-responsive inclinometer for indicating the angle of inclination of the needle relative to a patient's skin. It uses a plastic coupling tube for insertion between the syringe and needle of a conventional syringe and injection needle assembly and has means for mounting the inclinometer.

WO 2018/03860 A1 discloses an orientation indicator for a medicament delivery device comprising a support element, an activation member movable between a first position and a second position, an electrical circuit comprising a signal switch, a signalling element connected to the electrical circuit, and wherein the activation member may move under gravitational force when the support element is tilted, to actuate the signal switch, such that a signal may be generated by the signalling element.

US 2018/310956 A1 describes a system for guiding a needle into a target location of a patient's body. The system includes a planned needle guide trajectory and a guide for removable attachment to a smartphone. The guide includes at least one needle guiding aperture extending substantially perpendicularly to a screen of the smartphone. The needle guiding aperture defines an actual needle guide trajectory.

WO 2015/13883 A1 discloses a position guidance system for minimally invasive medical procedures including a medical device having a first end configured for percutaneous insertion and a second end configured to remain exterior to a patient's skin, a hub connection provided at the second end of the medical device, at least one bubble level including a bubble configured to provide a visual indication of a deviation from a target angle for medical device insertion, and a connector configured to reversibly and repeatedly connect the bubble level to the hub connection of the medical device. When the bubble is positioned at a centre of the bubble level, an actual insertion angle of the medical device is the same as the target angle. When the bubble is positioned off-centre of the bubble level, the actual insertion angle of the medical device deviates from the target angle.

EP 2 846 131 A1 discloses an inclinometer comprising a 2D-curved surface, a mobile element on said curved surface, being a ball, such that an inclination of the inclinometer causes a displacement of the mobile element on the curved surface, and a position sensor adapted to detect a position of said mobile element on said curved surface. Said curved surface is obtained by the deflection of a membrane under exposure to a differential pressure between one side of said membrane and the opposite side of said membrane, said opposite side being exposed to atmospheric pressure.

JP S52/114362A shows an inclinometer with a dome in which the angle can be read in 2D via markings within the dome.

### SUMMARY OF THE INVENTION

Based on US 4,479,800 as closest prior art relating to a devices to be used in a syringe and injection needle system, it is an object of the present invention to provide an improvement allowing the user to define and to read the 3D angle with respect to the perpendicular/vertical boundary line. In other words, a primary object of the invention is to provide an medical related instrument like an injecting needle or an Schanz pin with an inclinometer which will indicate to the user the angle of inclination of the needle relative to the patient's skin in two dimensions. Instead of one 3D angle, it could be mentioned that two or three 2D angles are to be read, or three Euler angles.

The gravity-responsive inclinometer for a medical device according to the present invention has a longitudinal coupling portion defining a predetermined device angle direction, wherein the longitudinal coupling portion comprises a connector part configured to be removably connected to the medical device, an at least partially transparent recipient, a ball and a measurement scale, wherein the ball is moving in the transparent recipient, indicating an angle vis-à-vis the predetermined angle directions through its position visible on the measurement scale. Said at least partially transparent recipient is a hollow transparent spherical cap attached at the connector part and the measurement scale comprises an indicator grid allowing a quick and correct determination of the angle of the axis of the medical device in the two directions vis-à-vis the plan of the surface of e.g. a patient relating to which the medical device has to be oriented. In this context it is an advantage, if the ball is translucent or transparent to be able to see the grid "through" the ball. The size of the ball can be chosen to have a diameter between 5 and 30% of the inner diameter of the transparent recipient, if this recipient is a sphere. That allows a good visible size and a weight which stay stable in view of the free-hand handling of the device.

The hollow transparent spherical cap of the inclinometer can be a hollow sphere or a hollow hemi-sphere with a cover, especially a flat cover. It is also possible that the spherical cap is comprises a different height between a full sphere and a partial dome with a height smaller than the radius of the sphere.

It can also be a hollow ellipsoid defined as a spheroid or a tri-axial ellipsoid. The grid can then still follow the great lines through the poles.

The main axes of the gird of the sphere do not need to be in line with the longitudinal axis of the coupling portion or in other words, the grid point, where the great-circles converge, usually, the point, where the ball would have it's 0 degree angle point is not necessarily this lowest point when the longitudinal axis of the coupling portion is vertical. Or, in other words, the 0 degree angle might show up (=ball at the "pole" grid intersection), when a specific inclination of the coupling portion is chosen and this is true for both angles deviating from the verticality.

The connector part of the longitudinal coupling portion can comprise a through-bore in the predetermined device angle direction for accommodating a coupling portion of the medical device. The connector part can then comprise a slit, especially opposite to the spherical cap in the predetermined device angle direction for clamping the coupling portion of the medical device in the through bore. A pole axis can be determined by real or virtual crossing points of the great-circle line portions, which pole axis is inclined in one or two directions, being orthogonal one to the other, vis-à-vis the longitudinal axis of the longitudinal coupling portion. The pole axis will usually have real parts, i.e. the actual grid, and will have virtual parts, .i.e. the prolongation of these grids which will not even join, if only a hemi-sphere is chosen as embodiment-

It is preferable that the indicator grid comprises at least one cross point allowing the user to establish an indicative measurement of the angular orientation of the medical device. It is then preferred, that the pole axis as cross point is a cross hair visualizing the pole position, especially for a translucent or transparent ball.

The connector part can then also comprise a slit in the predetermined device angle direction for clamping the coupling portion of the medical device in the through bore, i.e. to provide a force fit.

It is also possible that a lever-locked or a bayonet coupling is used but they are more complicated to mount; therefore clipping of the inclinometer on the medical device or introducing a longitudinal direction defining portion of the medical device into and through a through bore for a form or force fit are preferred embodiments.

The indicator grid preferredly comprises a plurality of great-circle (or orthodrome) line portions in the spherical cap crossing a plurality of small-circle line portions vertically, preferably in an angular distance of e.g. 30 degree one from the other.

The medical device can be an injection needle and syringe assembly. The medical device can be a Schanz pin, or any other medical device the user has to orient in a specific 3D angle vis-à-vis a surface.

The inclinometer can be provided to the user in sterilized condition.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a partially cut side view of a gravity-responsive inclinometer according to a first embodiment;
- Fig. 2: shows a look from above on the inclinometer of Fig. 1;
- Fig. 3: shows a partially cut side view of a gravity-responsive inclinometer according to a second embodiment;
- Fig. 4: shows a look from above on the inclinometer of Fig. 3;
- Fig. 5: shows a partially cut side view of a gravity-responsive inclinometer according to a third embodiment; and
- Fig. 6: shows a look from above on the inclinometer of Fig. 5;

### DESCRIPTION OF PREFERRED EMBODIMENTS

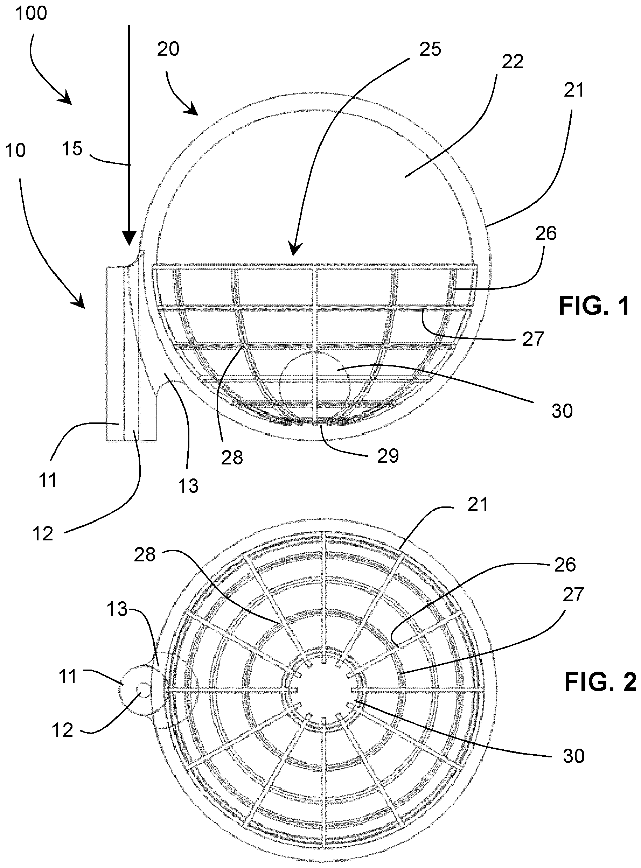

Fig. 1 shows a partially cut side view of a gravity-responsive inclinometer 10 according to a first embodiment, while Fig. 2 shows a look from above on the inclinometer of Fig. 1.

The gravity-responsive inclinometer 100 is intended to be used in connection with a medical device, here an injection needle and syringe assembly. The aim of the device is to know needle direction. This direction is defined by the direction of arrow 15 showing towards ground, i.e. providing a perpendicular direction which would be the predetermined device angle direction 15, when a medical device is attached at the longitudinal rod portion 10 of inclinometer 100.

The longitudinal rod portion 10 comprises a connector part configured to be removably connected to the medical device. The longitudinal rod portion 10 comprises a longitudinal cylinder wall 11 with an internal through bore 12 being in line with the longitudinal axis of the device to be attached, i.e. being in line with angle direction 15.

Said longitudinal rod portion 10 is connected with an at least partially transparent recipient 20 via an attachment portion 13. Thee attachment portion 13 can be made in one piece with the longitudinal rod portion 10. It is here only important, that the through going hollow bore 12, being the direction of axis 15, is oriented in a predetermined way with the measurement scale 25. It can be seen from Fig. 1 that each small circle line portion 25 defines a plan and these plans of small circle line portions 25 are parallel one to the other until the upper most portion 25 which is in fact a great circle line of the spherical hollow transparent recipient 20. Then the direction defining axis 15 of the medical device is perpendicular to the planes of the small circle line portions 25.

The hollow transparent recipient 20 is e.g. made of plastics which can be in one piece with the attachment portion 13 and the rod portion 10. Within surgical applications, the rod portion 10, the ball 30 as well as the recipient 20 are made from sterilizable material. Inside the inner hollow space 22 is provided a ball 30. Of course, this would necessitate a way to enter the ball 30 in space 22 during or prior to closure of transparent recipient 20. Preferably the ball has a diameter of between 10 and 52 per cent of the internal diameter of the hollow space 22. i.e. small enough not to cover too much lines of the measurement scale 25 but large enough for an easy positioning and recognition. Preferably the ball 30 is made of a heavy weight material as a steel ball with - at the same time - a small coefficient of friction against the plastics surface of the recipient 20 to allow the ball 30 to easily change position if the axis 15 is inclined in any way.

The transparent recipient 20 is a hollow sphere with a spherical inner surface on which the ball 30 is able to roll with small friction so that he is always ending his movement in the lowest position, when the device 100 is held still. Within the transparent recipient 20, preferably only in the lower half, a measurement scale is provided, indicating the angle vis-à-vis the predetermined angle direction 15 through its position visible on the measurement scale as position on or between scale lines 26 and 27. The measurement scale 25 comprises an indicator grid comprising a plurality of great-circle line portions 26 in the lower portion of the sphere 21 crossing a plurality of small-circle line portions 27 vertically at intersections 28.

The lines 27 and 28 can be laser printed lines, especially on the outside of the spherical cap 21. Then a user can identify the deviation of the position of the ball from the lowest point 29 in e.g. 15 or 30 degree steps, depending on the distance of the grid lines. Here the lowest point 29 is not indicated on the surface of the spherical cap 21, but could be a crosshair or any other identifiable element. In this context the lowest point 29 is at the same time the lowest cross point and the starting point of a pole axis which is here parallel to the longitudinal axis 15 of the longitudinal rod portion 11.

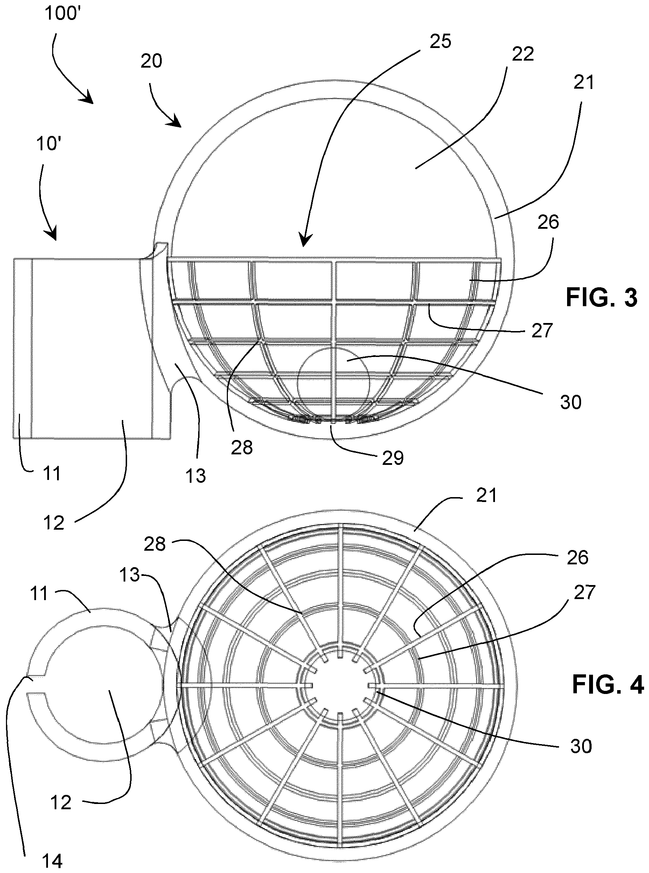

Fig. 3 shows a partially cut side view of a gravity-responsive inclinometer 100' according to a second embodiment; and Fig. 4 shows a look from above on the inclinometer 100' of Fig. 3. The transparent recipient, i.e. sphere 20 part is identical to the first embodiment. The difference is in the longitudinal rod portion 10', i.e. in the portion allowing to attach the inclinometer 100' to a medical device. Here the longitudinal rod portion 10' has a cylindrical wall 11 with a greater inner hollow diameter of the longitudinal through bore 12. The longitudinal through bore 12 of the first embodiment was small to allow a needle to be passed, whereas here a longitudinal slit 14 is provided allowing to clip a medical device with a slightly larger diameter than the longitudinal through bore 12 within the longitudinal rod portion 10'. The attachment portion 13 then connects this part of the device to the inclinometer measurement and showing part.

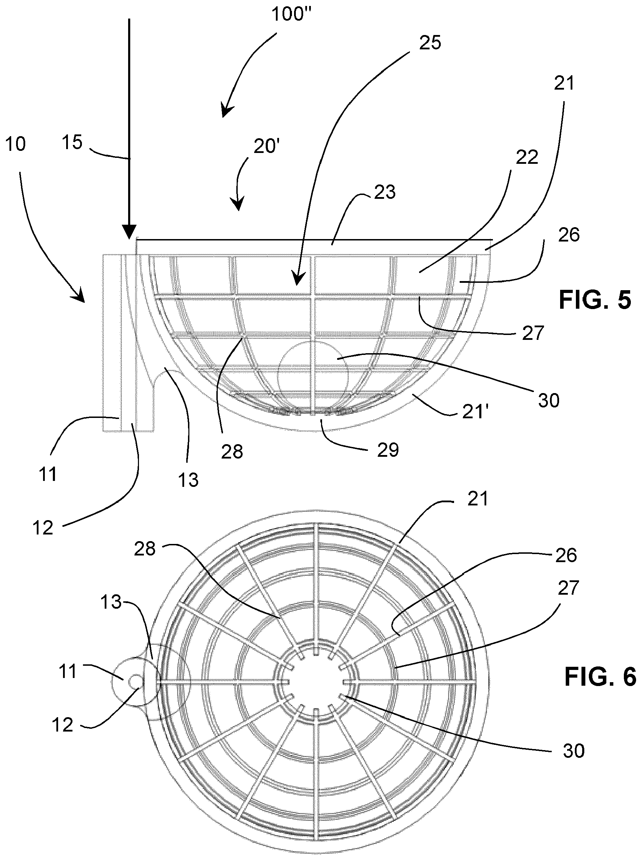

Fig. 5 shows a partially cut side view of a gravity-responsive inclinometer 100" according to a third embodiment; and Fig. 6 shows a look from above on the inclinometer 100" of Fig. 5. The longitudinal rod portion 10 of this third embodiment is as with the first embodiment, but could of course also be combined with the larger specimen of the second embodiment.

The main difference between the first two and this third embodiment is the transparent recipient 20' which is here a (in mathematical terms) spherical cap and especially a hemi-spherical cap. Usually a spherical cap is defined via the radius r of the sphere and either the height h of the cap or the base a of the cap or the polar angle to the apex of the cap (see https://en.wikipedia.org/wiki/Spherical cap). For the first two embodiments h = 2r, i.e. a full sphere while here h = r for a hemisphere. Therefore, the term spherical cap is used in the general case in the claims.

In Fig. 5 the inner hollow space 22 is covered by a flat lid 23 closing the hemisphere of the hemi-spherical cap 21'. Such a reduced place limits the possible movements of the ball 30 and would allow for a faster finding of the lowest position of the ball.

In none of the three embodiments, a specific handle is provided to hold the inclinometer. In the first and third embodiments, the user holds e.g. the syringe with a front plastic part or a needle going through the longitudinal rod portion 10, while in the second embodiment, the inclinometer 100' is clipped on the medical device. However, it is also possible that the inclinometer comprises a further handle element, especially, when the fit between the medical device and the inclinometer is not a force fit as in the second embodiment and/or a form fit as in the first or third embodiment or a combination but a locked connection as e.g. via a bayonet or levered connection.

**LIST OF REFERENCE SIGNS**

| | | | |
|---|---|---|---|
| 10 | longitudinal coupling portion | 23 | flat lid |
| 10' | longitudinal coupling portion | 25 | measurement scale |
| 11 | longitudinal cylinder wall | 26 | great-circle line portion |
| 12 | internal through bore | 27 | small-circle line portion |
| 13 | attachment portion | 28 | intersection |
| 14 | longitudinal slit | 29 | lowest point |
| 15 | device/syringe angle direction | 30 | ball |
| 20 | transparent recipient (sphere) | 100 | gravity-responsive inclinometer |
| 20' | transparent recipient (semispherical cap) | | |
| | | 100' | gravity-responsive |
| 21 | sphere | | inclinometer |
| 21' | spherical cap | 100" | gravity-responsive |
| 22 | hollow inner space | | inclinometer |

## Claims

1. A gravity-responsive inclinometer (100) for a medical device having a longitudinal coupling portion (10, 10') defining a predetermined device angle direction (15), wherein the longitudinal coupling portion (10, 10') comprises a connector part (11) configured to be removably connected to the medical device, an at least partially transparent recipient (20), a ball (30) and a measurement scale (25, 26, 27), wherein the ball (30) is moving in the transparent recipient (20), indicating an angle vis-à-vis the predetermined angle direction through its position visible on the measurement scale (25, 26, 27), **characterized in that**, that the transparent recipient (20) is a hollow (22) transparent spherical cap (21) attached (13) at the connector part and the measurement scale (25) comprises an indicator grid (26, 27).

2. The gravity-responsive inclinometer (100) according to claim 1, wherein the hollow transparent spherical cap is a hollow sphere (21), a hollow hemi-sphere (21) with a cover (23) or a hollow ellipsoid.

3. The gravity-responsive inclinometer (100) according to claim 1 or 2, wherein the indicator grid comprises a plurality of great-circle line portions (26) in the spherical cap (21) crossing a plurality of small-circle line portions (27) vertically.

4. The gravity-responsive inclinometer (100) according to claim 3, wherein a pole axis is determined by real or virtual crossing points of the great-circle line portions (26), which pole axis is parallel to the longitudinal axis (15) of the longitudinal coupling portion (10, 10').

5. The gravity-responsive inclinometer (100) according to claim 3, wherein a pole axis is determined by real or virtual crossing points of the great-circle line portions (26), which pole axis is inclined in one or two directions, being orthogonal one to the other, vis-à-vis the longitudinal axis (15) of the longitudinal coupling portion (10, 10').

6. The gravity-responsive inclinometer (100) according to any one of claims 1 to 5, wherein the connector part (11) of the longitudinal coupling portion (10) comprises a through-bore (12) in the predetermined device angle direction (15) for accommodating a coupling portion of the medical device.

7. The gravity-responsive inclinometer (100) according to claim 6, wherein the connector part (11) comprises a slit (14) in the predetermined device angle direction (15) for clamping the coupling portion of the medical device in the through bore (12).

8. The gravity-responsive inclinometer (100) according to any one of claims 1 to 7, wherein the medical device is an injection needle and syringe assembly or a Schanz pin.

9. The gravity-responsive inclinometer (100) according to any one of claims 1 to 8, wherein the inclinometer (100) is provided in sterilized condition.

10. The gravity-responsive inclinometer (100) according to any one of claims 1 to 9, wherein the indicator grid (26, 27) comprises at least one cross point (28).

11. The gravity-responsive inclinometer (100) according to claim 3 or 4 and claim 10, wherein the pole axis as cross point is a cross hair visualizing the pole position, especially for a translucent or transparent ball (30).
